# EUROPÄISCHE PATENTANMELDUNG

(11) **EP 1 576 946 A1**
(43) Veröffentlichungstag der Anmeldung: **21.09.2005**
(21) Anmeldenummer: 05005720.7
(22) Anmeldetag: 16.03.2005
(51) Int. Cl.: A61K 7/32, A61K 7/36, A61K 7/38

(54) **Verwendung von Inhibitoren Gram-positiver Kokken in Deodorantien und Antitranspirantien**

(30) Priorität: 18.03.2004 DE 102004013695; 03.07.2004 DE 102004032265
(71) Anmelder: Henkel Kommanditgesellschaft auf Aktien, 40589 Düsseldorf (DE)
(72) Erfinder: Banowski, Bernhard, 40597 Düsseldorf (DE); Bockmühl, Dirk, 42113 Wuppertal (DE); Scholtyssek, Regine, 40882 Mettmann (DE)

(57) **Zusammenfassung**

Gegenstand der Erfindung ist die Verwendung von mindestens einer Substanz, die an der Körpergeruchsbildung beteiligte Gram-positive anaerobe Kokken inhibiert, in einer kosmetischen Deodorant- oder Antitranspirant-Zusammensetzung zur Verringerung des Körpergeruchs.

## Beschreibung

Gegenstand der Erfindung ist die Verwendung von ausgewählten Substanzen, welche Gram-positive anaerobe Kokken inhibieren, in einer kosmetischen Deodorant- oder Antitranspirant-Zusammensetzung zur Verringerung des Körpergeruchs.

Apokriner Schweiß stellt eine komplexe Mischung dar, die unter anderem Steroide, Cholesterin und andere Fette sowie ca. 10 % Eiweiße enthält. Körpergeruch entsteht durch den Abbau von Schweißbestandteilen durch Bakterien der Hautflora. Aus diesem Grund werden seit langem antibakterielle Substanzen in Deos verwendet. Allerdings ist bekannt, dass viele antibakterielle Wirkstoffe schlecht gegen Körpergeruch wirken. Die an der Geruchsbildung beteiligten Bakterienarten werden üblicherweise über die Kultivierung von Achselproben auf Bakteriennährböden nachgewiesen. Diese Methode hat den Nachteil, dass nicht oder nur schwer kultivierbare Arten, die an der Entstehung von Körpergeruch beteiligt sind, nicht erfasst werden. Folglich sind derartige Spezies auch nicht das Ziel von Versuchen zur Bewertung der Deodorantleistung. Außerdem lässt sich auch hinsichtlich der kultivierbaren Arten keine zuverlässige Aussage zur Menge der vorhandenen Keime treffen, da einige Arten sich besser kultivieren lassen als andere, so dass keine Aussage bezüglich ihrer Bedeutung mit Hinblick auf die Entstehung von Körpergeruch getroffen werden kann. Infolgedessen kommt es zum Einsatz vermeintlich antibakterieller Substanzen, die jedoch aufgrund des falschen Wirkspektrums nur eine unzureichende Deodorantleistung besitzen.

Die Zersetzungsprodukte des apokrinen Schweißes, die wesentlich zum Körpergeruch, insbesondere zum axillaren Körpergeruch, beitragen, lassen sich in zwei Klassen einteilen, zum einen kurzkettige, insbesondere C₄-C₁₀-Fettsäuren, die linear, verzweigt, gesättigt und ungesättigt sein können, zum anderen verschiedene Steroidhormone und deren Stoffwechselprodukte. Beispielsweise sind an dem typischen Körpergeruch, besonders an dem der Männer, die Stoffwechselprodukte der Androgene beteiligt, insbesondere Androstenol (5α-Androst-16-en-3β-ol, 5α-Androst-16-en-3α-ol) und Androstenon (5α-Androst-16-en-3-on).

Aufgabe der vorliegenden Erfindung war es, weitere Bakterien-inhibierende Wirkstoffe zu identifizieren, um eine größere Variabilität, Flexibilität und Hautverträglichkeit bei der Formulierung kosmetischer Deodorantien zu ermöglichen. Die Identifizierung bekannter kosmetischer Wirkstoffe als Inhibitoren für bestimmte geruchsbildende Bakterien ermöglicht darüber hinaus, die Dosierung dieser Wirkstoffe herunterzusetzen.

Es wurde nun gefunden, dass der Hemmung Gram-positiver anaerober Kokken bei der Bekämpfung von Körpergeruch eine besondere Bedeutung zukommt.

Gegenstand der vorliegenden Erfindung ist in einer ersten Ausführungsform die Verwendung von mindestens einer Substanz, die an der Körpergeruchsbildung beteiligte Gram-positive anaerobe Kokken inhibiert, in einer kosmetischen Deodorant- oder Antitranspirant-Zusammensetzung zur Verringerung des Körpergeruchs.

Zu den Kokken zählen die Familie I (Veillonella) mit den Gattungen Veillonella sowie die Familie II (Peptococcacaeae) mit den Gattungen Peptococcus und Peptostreptococcus. Gram-positive Kokken lassen sich in die Familie der Micrococcaceae (Gattungen: Micrococcus, Stomatococcus, Planococcus, Staphylococcus) und die Familie der Deinococcaceae (Gattung: Deinococcus andere Organismen: z.B. Streptococci) einteilen.

Besonders bevorzugt ist es erfindungsgemäß, wenn Bakterien der Gattung *Peptostreptococcus* inhibiert werden. Eine erfindungsgemäße Verwendung, die dadurch gekennzeichnet ist, dass die an der Körpergeruchsbildung beteiligten Gram-positiven anaeroben Kokken ausgewählt sind aus Bakterien der Gattung *Peptostreptococcus,* ist demnach besonders bevorzugt.

Die Gattungsbezeichnung Peptostreptococcus beinhaltet die Gattungssynonyme *Peptoniphilus, Gallicola, Siackia, Anaerococcus (u.a. Anaerococcus octavius), Finegoldia, Micromonas, Atopobium* sowie *Ruminococcus.* Die vorstehend genannten erfindungsgemäß bevorzugten Verwendungen sind daher dadurch gekennzeichnet, dass die an der Körpergeruchsbildung beteiligten Gram-positiven anaeroben Kokken ausgewählt sind aus Bakterien der Gattung *Peptoniphilus, Gallicola, Siackia, Anaerococcus (u.a. Anaerococcus octavius), Finegoldia, Micromonas, Atopobium* sowie *Ruminococcus.*

Weitere bevorzugte erfindungsgemäße Verwendungen sind dadurch gekennzeichnet, dass mindestens eine Bakterien der Gattung *Peptostreptococcus* inhibierende Substanz insbesondere *Anaerococcus octavius,* das auch als *Peptostreptococcus octavius* bezeichnet wird, inhibiert.

Unter den die an der Körpergeruchsbildung beteiligten Gram-positiven anaeroben Kokken inhibierenden Substanzen werden erfindungsgemäß solche Substanzen verstanden, deren Wirkung zu einer statistisch signifikanten Verlangsamung oder völligen Hemmung des mikrobiellen Wachstums der Gram-positiven anaeroben Kokken im Vergleich zu einer unbehandelten Kontrolle führt.

Als Substanzen, die die an der Körpergeruchsbildung beteiligten Gram-positiven Kokken inhibieren und erfindungsgemäß in einer kosmetischen Deodorant- oder Antitranspirant-Zusammensetzung zur Verringerung des Körpergeruchs eingesetzt werden können, eignet sich eine Vielzahl von Verbindungen. Besonders bevorzugte erfindungsgemäße Verwendungen sind dadurch gekennzeichnet, dass die an der Körpergeruchsbildung beteiligte Gram-positive anaerobe Kokken und insbesondere Bakterien der Gattung *Peptostreptococcus* inhibierende(n) Substanz(en) ausgewählt ist/sind aus funktionellen C₂-C₁₂-Carbonsäuren und deren physiologisch verträglichen Metallsalzen, einfach ungesättigten cis-C₄-C₃₀-Dicarbonsäuren, Derivaten von C₃-C₉-Polyolen, Phenolderivaten, primären Alkanolen mit einer Alkylkettenlänge von C₃ - C₈ und einem Benzyl- oder Phenylsubstituenten, Aluminiumchlorohydrat, Aluminiumzirkoniumchlorohydrat und den Riechstoffgemischen Protectate HR und Protectate MOD 2 der Firma Symrise sowie Mischungen dieser Substanzen.

In einer bevorzugten Ausführungsform sind die erfindungsgemäß verwendeten funktionellen C₂-C₁₂-Carbonsäuren ausgewählt aus C₂-C₁₂-Hydroxycarbonsäuren, C₃-C₁₂-Ketocarbonsäuren, C₂-C₅-Aminosäuren und deren N-C₂-C₁₂-Acylderivaten sowie den physiologisch verträglichen Metallsalzen dieser Säuren.

Die physiologisch verträglichen Metallsalze sind ausgewählt aus den Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.

Erfindungsgemäß werden die funktionellen C₂-C₁₂-Carbonsäuren und ihre physiologisch verträglichen Metallsalze in Gesamtmengen von 0,001 bis 10 Gew.-%, bevorzugt 0,005 bis 5 Gew.-% und besonders bevorzugt 0,01 bis 2 Gew.-%, jeweils bezogen auf die gesamte kosmetische Zusammensetzung, eingesetzt.

Erfindungsgemäß bevorzugt verwendete C₂-C₁₂-Hydroxycarbonsäuren und ihre physiologisch verträglichen Metallsalze sind ausgewählt aus den alpha-Monohydroxy-C₃-C₁₂-Carbonsäuren und ihren Salzen, bevorzugt Milchsäure, Aluminiumlactat, Zinklactat, alpha-Hydroxycaprinsäure, alpha-Hydroxycaprylsäure und alpha-Hydroxylaurinsäure, sowie den Polyhydroxy-C₃-C₈-Carbonsäuren, bevorzugt D-Gluconsäure, Aluminiumgluconat, Zinkgluconat, Mangangluconat, D-Glucoheptonsäure und Magnesiumglucoheptonat. Zinkgluconat ist erfindungsgemäß besonders bevorzugt.

Erfindungsgemäß bevorzugt verwendete C₃-C₁₂-Ketocarbonsäuren sind beispielsweise Brenztraubensäure, Acetessigsäure und Lävulinsäure (4-Oxopentansäure). Besonders bevorzugt ist Lävulinsäure.

Erfindungsgemäß bevorzugt verwendete C₂-C₅-Aminosäuren, deren N-C₂-C₁₂-Acylderivate sowie die physiologisch verträglichen Metallsalze der genannten Verbindungen sind beispielsweise Glycin, Aluminiumglycinat, Alanin, Glutaminsäure, Pyroglutaminsäure, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Aluminiumglycinat, Zinkglycinat, Zinkpyroglutamat und Natriumlauroylglutamat.

Wie vorstehend erwähnt, lassen sich auch einfach ungesättigte cis-C₄-C₃₀-Dicarbonsäuren erfindungsgemäß einsetzen. Bevorzugte Spezies aus dieser Gruppe sind unverzweigt und lassen sich durch die Formel

H₃C-(CH₂)ₙ-CH=CH-(CH₂)ₘ-CH₃

beschreiben, in der n + m = 0 bis 26 gilt. In besonders bevorzugten erfindungsgemäßen Verwendungen sind die einfach ungesättigten cis-C₄-C₃₀-Dicarbonsäuren ausgewählt aus 5(Z)-Decendicarbonsäure, 6(Z)-Dodecendicarbonsäure, 7(Z)-Tetradecendicarbonsäure, 8(Z)-Hexadecendicarbonsäure, 9(Z)-Octadecendicarbonsäure und 10(Z)-Eicosendicarbonsäure. Besonders bevorzugt ist 9(Z)-Octadecendicarbonsäure, erhältlich z. B. von Uniqema unter der Bezeichnung Arlatone DCA.

Erfindungsgemäß werden die C₂-C₁₂-Hydroxycarbonsäuren, die C₃-C₁₂-Ketocarbonsäuren, die C₂-C₅-Aminosäuren, deren N-C₂-C₁₂-Acylderivate sowie einfach ungesättigte cis-C₄-C₃₀-Dicarbonsäuren und die jeweiligen physiologisch verträglichen Metallsalze in Gesamtmengen von 0,001 bis 10 Gew.-%, bevorzugt 0,005 bis 5 Gew.-% und besonders bevorzugt 0,01 bis 2 Gew.-%, jeweils bezogen auf die gesamte kosmetische Zusammensetzung, eingesetzt.

Erfindungsgemäß bevorzugt verwendete Derivate von C₃-C₉-Polyolen sind ausgewählt aus Ascorbinsäure, Monoalkyl-C₃-C₉-Polyolethern, Ethern und Estern von gewünschtenfalls alkylierter Glucose oder Oligoglucose sowie den Tetraestern von Pentaerythrit mit einen substituierten Arylrest enthaltenden C₂-C₄-Carbonsäuren.
Bevorzugte erfindungsgemäße Verwendungen sind dadurch gekennzeichnet, dass die Derivate von C₃-C₉-Polyolen ausgewählt sind aus Monoalkyl-C₃-C₉-Polyolethern. Bevorzugt verwendete Monoalkyl-C₃-C₉-Polyolether sind insbesondere Monoalkylglycerinether der allgemeinen Formel R-O-CH₂-CHOH-CH₂OH, wobei R eine lineare oder verzweigte C₁-C₂₂-Alkylgruppe darstellt. R ist bevorzugt eine C₆-C₁₂-Alkylgruppe, die in 2-Position eine weitere Alkylgruppe, bevorzugt eine Methyl-, Ethyl- oder n-Propylgruppe aufweist. Besonders bevorzugt sind a-Monohexylglycerinether, α-Mono-(2-ethylhexyl)glycerinether, a-Mono-(2-ethyloctyl)glycerinether und a-Mono-(2-ethyldecyl)glycerinether. Außerordentlich bevorzugt ist α-Mono-(2-ethylhexyl)glycerinether oder kurz 2-Ethylhexylglycerinether, erhältlich unter dem Namen Sensiva SC 50 von Schülke und Mayr.

Ein bevorzugt verwendeter Glucoseether ist Ascorbylglucosid.
Weiterhin werden als Ether von Glucose oder Oligoglucose C₈ - C₂₂-Alkylmono- und -oligoglucoside bevorzugt verwendet. C₈ -C₂₂-Alkylmono- und -oligoglucoside stellen bekannte, handelsübliche Tenside und Emulgatoren dar. Ihre Herstellung erfolgt insbesondere durch Umsetzung von Glucose mit primären Alkoholen mit 8 - 22 Kohlenstoffatomen. Die erfindungsgemäß verwendeten C₈₋₂₂-Alkylmono- und -oligoglucoside entsprechend der allgemeinen Formel RO-(G)ₓ, wobei R für eine C₈-C₂₂-Alkylgruppe, G für Glucose sowie x, die Anzahl der Glucoseeinheiten, für eine rationale Zahl von 1 - 8 steht, sind bevorzugt solche, bei denen R im wesentlichen aus C₈- und C₁₀-Alkylgruppen, im wesentlichen aus C₁₂- und C₁₄-Alkylgruppen, im wesentlichen aus C₈- bis C₁₆-Alkylgruppen, im wesentlichen aus C₁₆- bis C₂₂-Alkylgruppen oder im wesentlichen aus C₁₂- bis C₁₆-Alkylgruppen besteht. Bezüglich des Glucosidrestes gilt, dass sowohl Monoglucoside, bei denen ein cyclischer Glucoserest glycosidisch an den Fettalkohol gebunden ist, als auch oligomere Glucoside mit einem Oligomerisationsgrad von 1 bis etwa 8, vorzugsweise 1 - 2, besonders bevorzugt 1, 1,1 , 1,2 , 1,3 , 1,4 , 1,5 und 1,6, geeignet sind. Der Oligomerisierungsgrad ist dabei ein statistischer Mittelwert, dem eine für solche technischen Produkte übliche Homologenverteilung zugrunde liegt. Produkte, die z. B. unter dem Handelsnamen Plantacare® oder Plantaren® erhältlich sind, enthalten eine glucosidisch gebundene C₈-C₂₂-Alkylgruppe an einem Oligoglucosidrest mit einem mittleren Oligomerisationsgrad von 1 - 2. Besonders bevorzugt ist die Verwendung eines C₈₋₁₆-Alkyloligoglucosids mit der Bezeichnung Plantaren® 2000 mit der INCI-Bezeichnung Decyl Glucoside.

Bevorzugt verwendete Glucoseester sind die Ester von hydroxysubstituierten Bi- oder Tricarbonsäuren mit alkylierter Glucose der allgemeinen Formel (I), 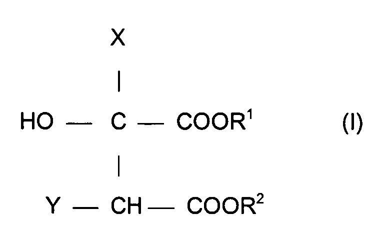 in der X ein Wasserstoff-Atom oder eine -CH₂COOR-Gruppe ist, Y ein Wasserstoff-Atom oder -OH-Gruppe ist unter der Bedingung, dass Y ein Wasserstoff-Atom ist, wenn X -CH₂COOR ist, R, R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, wobei Z einen mit einem C₆-C₁₈-Alkanol oder mit technischen Mischungen von C₆-C₁₈-Alkanolen veretherten Glucose- oder Oligoglucoserest darstellt, mit der Bedingung, dass mindestens eine der Gruppen R, R¹ oder R² ein Rest Z ist. Solche Verbindungen sind z. B. in der Druckschrift EP 258 814 B1 beschrieben. Besonders bevorzugt verwendete Verbindungen der Formel (I) sind Dinatrium-Kokosalkylpolyglucosidcitrat (Disodium Cocopolyglucose Citrate, als Handelsprodukt Eucarol® AGE EC von der Firma Cesalpinia Chemicals erhältlich) und Natrium-Kokosalkylpolyglucosidtartrat (Sodium Cocopolyglucose Tartrate, als Handelsprodukt Eucarol® AGE ET von der Firma Cesalpinia Chemicals erhältlich).
Bevorzugt verwendete Tetraester von Pentaerythrit mit einen substituierten Arylrest enthaltenden C₂-C₄-Carbonsäuren sind abgeleitet von Essigsäure, die am C2-Atom einen substituierten Arylrest aufweist, Propionsäure, die am C3-Atom einen substituierten Arylrest aufweist und Buttersäure, die am C4-Atom einen substituierten Arylrest aufweist. Die substituierten Arylreste weisen bevorzugt in 4-Stellung eine Hydroxylgruppe sowie in 3- und/oder 5-Stellung eine C₁-C₄-Alkylgruppe auf. Diese C₁-C₄-Alkylgruppen sind ausgewählt aus Methyl-, Ethyl-, n-Propyl-, Isopropyl, n-Butyl-, sec-Butyl- und tert-Butyl-Gruppen. Eine besonders bevorzugt verwendete Verbindung dieses Typs ist Pentaerythrittetrakis[3(3,5-di-tert.-butyl-4-hydroxyphenyl)propionat], als Handelsprodukt Tinogard® TT DD von Ciba erhältlich.
Erfindungsgemäß werden die Derivate von C₃-C₉-Polyolen in Gesamtmengen von 0,01 bis 15 Gew.-%, bevorzugt 0,05 bis 10 Gew.-% und besonders bevorzugt 0,1 bis 5 Gew.-%, jeweils bezogen auf die gesamte kosmetische Zusammensetzung, eingesetzt.
Erfindungsgemäß werden die Derivate von C₃-C₉-Polyolen, die ausgewählt sind aus Monoalkylglycerinethern der allgemeinen Formel R-O-CH₂-CHOH-CH₂OH, in Gesamtmengen von 0,01 bis 10 Gew.-%, bevorzugt 0,05 bis 5 Gew.-% und besonders bevorzugt 0,1 bis 3 Gew.-%, jeweils bezogen auf die gesamte kosmetische Zusammensetzung, eingesetzt.

Erfindungsgemäß bevorzugt verwendete Phenolderivate sind ausgewählt aus Phenoxyethanol, 4-Methoxybenzylalkohol (para-Anisalkohol), 5-Methyl-2-isopropylphenol (Thymol), Salicylsäure, Rosmarinsäure, Kaffeesäure, Tocopherolen und Tocopherylestern, insbesondere α-Tocopherol, Tocopherylacetat, Tocopherylnicotinat, Tocopherylphosphat und Tocopherylsuccinat, Benzotriazolyl-substituierten 4-Phenolsulfonsäuren und deren Salzen, Polyphenolen sowie Mischungen der vorgenannten Substanzen.
Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho und Eichenrinde sowie anderer Baumrinden, Blättern von Grünem Tee (camellia sinensis) und Mate. Ebenfalls besonders bevorzugt sind die Tannine.
Die bevorzugt verwendeten Benzotriazolyl-substituierten 4-Phenolsulfonsäuren und ihre Salze enthalten den Benzotriazolyl-Rest in 3-Position. Besonders bevorzugt werden solche Verbindungen verwendet, die zusätzlich in der 5-Position eine C₁-C₄-Alkylgruppe aufweisen. Die C₁-C₄-Alkylgruppen sind dabei ausgewählt aus Methyl-, Ethyl-, n-Propyl-, Isopropyl-, n-Butyl-, sec-Butyl- und tert-Butyl-Gruppen. Erfindungsgemäß geeignete Salze der 4-Phenolsulfonsäuren sind alle mit physiologisch verträglichen Kationen, beispielsweise Natrium, Kalium und Ammonium. Besonders bevorzugt verwendet wird das 3-Benzotriazolyl-5-sec-butyl-4-phenolsulfonsäure-natriumsalz, als Handelsprodukte Tinogard™ HS (Reinsubstanz) oder Cibafast™ H (Compound mit Buteth-3 und Tributylcitrat) von Ciba erhältlich.

Besonders bevorzugt sind außerdem Phenoxyethanol, 4-Methoxybenzylalkohol (para-Anisalkohol) und 5-Methyl-2-isopropylphenol (Thymol) sowie Mischungen dieser Substanzen.
Erfindungsgemäß werden die Phenolderivate in Gesamtmengen von 0,001 bis 10 Gew.-%, bevorzugt 0,005 bis 5 Gew.-% und besonders bevorzugt 0,08 bis 3 Gew.-%, jeweils bezogen auf die gesamte kosmetische Zusammensetzung, eingesetzt.

Erfindungsgemäß bevorzugt verwendete primäre Alkanole mit einer Alkylkettenlänge von C₃ - C₈ und einem Benzyl- oder Phenylsubstituenten weisen n-Propanol-1, n-Butanol-1, n-Pentanol-1, n-Hexanol-1, n-Heptanol-1 oder n-Octanol-1 als Grundgerüst auf, das mit Methyl-, Ethyl-, n-Propyl-, iso-Propyl-, n-Butyl-, sec-Butyl- oder tert.-Butyl-Gruppen verzweigt sein kann. Weiterhin enthält das Gründgerüst in 2-Stellung, 3-Stellung, 4-Stellung, 5-Stellung, 6-Stellung, 7-Stellung oder 8-Stellung eine Benzyl- oder Phenylgruppe, die gegebenenfalls weitere Substituenten aufweisen kann, beispielsweise Methyl-, Ethyl-, n-Propyl-, iso-Propyl-, n-Butyl-, sec-Butyl- oder tert.-Butyl-Gruppen, Hydroxylgruppen oder Halogenatome, insbesondere Fluor und Chlor.
Besonders bevorzugte primäre Alkanole mit einer Alkylkettenlänge von C₃ - C₈ und einem Benzyl- oder Phenylsubstituenten sind ausgewählt aus 2-Methyl-5-phenylpentan-1-ol (mit dem Trivialnamen Rosaphen) und 2-Benzylheptan-1-ol (mit dem Trivialnamen Jasmol).
Erfindungsgemäß werden die primären Alkanole mit einer Alkylkettenlänge von C₃ - C₈ und einem Benzyl- oder Phenylsubstituenten in Gesamtmengen von 0,001 bis 10 Gew.-%, bevorzugt 0,005 bis 5 Gew.-% und besonders bevorzugt 0,03 bis 1,0 Gew.-%, jeweils bezogen auf die gesamte kosmetische Zusammensetzung, eingesetzt.

Erfindungsgemäß bevorzugt ist die Verwendung von Aluminiumchlorohydraten, insbesondere Aluminiumchlorohydrat und/oder Aluminiumzirkoniumchlorohydrat. Aluminiumchlorohydrat ist ein gebräuchlicher Antitranspirant-Wirkstoff, der als Adstringens allerdings in höheren Konzentrationen von 5 bis 25 Gew.-% eingesetzt werden muss, um in für den Verbraucher akzeptabler Weise wirksam zu sein. Für die Inhibierung von Gram-positiven anaeroben Kokken genügen dagegen niedrigere Konzentrationen von 0,2 bis 3 Gew.-%, bevorzugt 0,5 bis 3 Gew.-% und besonders bevorzugt 1,0 bis 2,0 Gew.-%, jeweils bezogen auf die gesamte kosmetische Zusammensetzung.

Erfindungsgemäß wird Aluminiumchlorohydrat und/oder Aluminiumzirkoniumchlorohydrat in Mengen von 0,1 bis 25 Gew.-%, bevorzugt 0,5 bis 10,0 und besonders bevorzugt 1,0 bis 5,0 Gew.-%, jeweils bezogen auf die gesamte kosmetische Zusammensetzung, eingesetzt. Antitranspirantprodukte mit einem hohen Wirkstoffanteil von bis zu 25 Gew.% besitzen auch diese Eigenschaft.

Das erfindungsgemäß bevorzugte Riechstoffgemisch Protectate HR der Firma Symrise enthält 25 - 50 Gew.-% Phenoxyethanol, 5 - 10 Gew.-% 2-Methyl-5-phenylpentan-1-ol mit dem Trivialnamen Rosaphen, 34 - 70 Gew.-% 2-Benzylheptan-1-ol mit dem Trivialnamen Jasmol, 1 - 5 Gew.-% 4-Methoxybenzylalkohol (Anisalkohol) und 0,01 - 1 Gew.-% 5-Methyl-2-isopropylphenol (Thymol). Das erfindungsgemäß bevorzugte Riechstoffgemisch Protectate MOD 2 der Firma Symrise enthält 25 - 45 Gew.-% Phenoxyethanol, 5 - 10 Gew.-% 2-Methyl-5-phenylpentan-1-ol und 45 - 70 Gew.-% 2-Benzyl-heptan-1-ol.

Erfindungsgemäß werden die Riechstoffgemische Protectate HR oder Protectate Mod 2 in Gesamtmengen von 0,001 bis 5 Gew.-%, bevorzugt 0,05 bis 2 Gew.-% und besonders bevorzugt 0,1 bis 1,0 Gew.-%, jeweils bezogen auf die gesamte kosmetische Zusammensetzung, eingesetzt.

Neben der Verwendung der genannten Einzelverbindungen ist die Verwendung von Kombinationen von zwei, drei und mehr Inhibitoren von an der Körpergeruchsbildung beteiligten Gram-positiven anaeroben Kokken, insbesondere Bakterien der Gattung *Peptostreptococcus,* erfindungsgemäß besonders bevorzugt.
Besonders bevorzugt sind Mischungen aus 0,1 - 2,0 Gew.-%, insbesondere 0,4 Gew.-% α-Mono-(2-ethylhexyl)glycerinether, 0,1 - 2,5 Gew.-%, insbesondere 0,8 Gew.-% Phenoxyethanol und 0,05 - 1,0 Gew.-%, insbesondere 0,2 Gew.-% Protectate MOD 2, jeweils bezogen auf das Gewicht der gesamten kosmetischen Zusammensetzung.

Weiterhin wurde gefunden, dass ein Gehalt an Triethylcitrat die Wirkung der vorgenannten Substanzen auf die Inhibierung von an der Körpergeruchsbildung beteiligten Gram-positiven anaeroben Kokken, insbesondere Bakterien der Gattung *Peptostreptococcus,* weiter steigern kann. Damit ist die Verwendung von Mischungen aus 0,1 - 1,0 Gew.-% α-Mono-(2-ethylhexyl)glycerinether, 0,1 - 2,0 Gew.-% Phenoxyethanol, 0,05 - 1,0 Gew.-% Protectate MOD 2 und 0,1 - 6 Gew.-%, insbesondere 0,5 - 4 Gew.-% Triethylcitrat, jeweils bezogen auf das Gewicht der gesamten kosmetischen Zusammensetzung, erfindungsgemäß besonders bevorzugt.

Ein weiterer Gegenstand der vorliegenden Erfindung ist ein Verfahren zur Verringerung von Körpergeruch mittels Inhibierung von an der Körpergeruchsbildung beteiligten Gram-positiven anaeroben Kokken, insbesondere Bakterien der Gattung *Peptostreptococcus,* auf der Haut, dadurch gekennzeichnet, dass eine kosmetische Deodorant- oder Antitranspirant-Zusammensetzung, enthaltend mindestens eine an der Körpergeruchsbildung beteiligte Gram-positive anaerobe Kokken, insbesondere Bakterien der Gattung *Peptostreptococcus* inhibierende Substanz auf die Haut, insbesondere auf die Haut der Achselhöhlen, aufgetragen wird.

Bevorzugte erfindungsgemäße Verfahren sind dadurch gekennzeichnet, dass die an der Körpergeruchsbildung beteiligte(n) Gram-positive anaerobe Kokken, insbesondere Bakterien der Gattung *Peptostreptococcus* inhibierende(n) Substanz(en) ausgewählt ist/sind aus funktionellen C₂-C₁₂-Carbonsäuren und deren physiologisch verträglichen Metallsalzen, einfach ungesättigten cis-C₄-C₃₀-Dicarbonsäuren, Derivaten von C₃-C₉-Polyolen, Phenolderivaten, primären Alkanolen mit einer Alkylkettenlänge von C₃ - C₈ und einem Benzyl- oder Phenylsubstituenten, Aluminiumchlorohydrat, Aluminiumzirkoniumchlorohydrat und den Riechstoffgemischen Protectate HR und Protectate MOD 2 der Firma Symrise sowie Mischungen dieser Substanzen.

Die kosmetischen Deodorant- oder Antitranspirant-Zusammensetzungen, die die erfindungsgemäß verwendeten Inhibitoren für Gram-positive Kokken, insbesondere *Peptostreptococcus*, enthalten, können als Puder, in Stiftform, als Aerosolspray, Pumpspray, flüssige und gelförmige Roll on-Applikation, Creme, Gel und als getränktes flexibles Substrat vorliegen.
Deodorant- oder Antitranspirant-Stifte können in gelierter Form, auf wasserfreier Wachsbasis und auf Basis von W/O-Emulsionen und O/W-Emulsionen vorliegen. Gelstifte können auf der Basis von Fettsäureseifen, Dibenzylidensorbitol, N-Acylaminosäureamiden, 12-Hydroxystearinsäure und anderen Gelbildnern hergestellt werden.
Aerosolsprays, Pumpsprays, Roll on-Applikationen und Cremes können als Wasser-in-Öl-Emulsion, Öl-in-Wasser-Emulsion, Siliconöl-in-Wasser-Emulsion, Wasser-in-Siliconöl-Emulsion, Wasser-in-Öl-Mikroemulsion, Öl-in-Wasser-Mikroemulsion, Siliconöl-in-Wasser-Mikroemulsion, Wasser-in-Siliconöl-Mikroemulsion, wasserfreie Suspension, alkoholische und hydroalkoholische Lösung, wässriges Gel und als Öl vorliegen. Alle genannten Zusammensetzungen können verdickt sein, beispielsweise auf der Basis von Fettsäureseifen, Dibenzylidensorbitol, N-Acylaminosäureamiden, 12-Hydroxystearinsäure, Polyacrylaten vom Carbomer- und Carbopol-Typ, Polyacrylamiden und Polysacchariden, die chemisch und/oder physikalisch modifiziert sein können.
Die Emulsionen und Mikroemulsionen können transparent, translucent oder opak sein.

Die kosmetischen Deodorant- oder Antitranspirant-Zusammensetzungen, die die erfindungsgemäß verwendeten Inhibitoren für Gram-positive Kokken, insbesondere *Peptostreptococcus*, enthalten, können weiterhin Fettstoffe enthalten. Unter Fettstoffen sind Fettsäuren, Fettalkohole, natürliche und synthetische kosmetische Ölkomponenten sowie natürliche und synthetische Wachse zu verstehen, die sowohl in fester Form als auch flüssig in wässriger oder öliger Dispersion vorliegen können.
Als Fettsäuren können eingesetzt werden lineare und/oder verzweigte, gesättigte und/oder ungesättigte C₈₋₃₀-Fettsäuren. Bevorzugt sind C₁₀₋₂₂-Fettsäuren. Beispiele sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachidonsäure, Gadoleinsäure, Behensäure und Erucasäure sowie deren technische Mischungen. Besonders bevorzugt ist der Einsatz von Stearinsäure. Die eingesetzten Fettsäuren können eine oder mehrere Hydroxygruppen tragen. Bevorzugte Beispiele hierfür sind die α-Hydroxy-C₈-C₁₈-Carbonsäuren sowie 12-Hydroxystearinsäure.
Die Einsatzmenge beträgt dabei 0,1 - 15 Gew.-%, bevorzugt 0,5 - 10 Gew.-%, besonders bevorzugt 1 - 7 Gew.-%, jeweils bezogen auf die gesamte Zusammensetzung.

Als Fettalkohole können eingesetzt werden gesättigte, ein- oder mehrfach ungesättigte, verzweigte oder unverzweigte Fettalkohole mit 6 - 30, bevorzugt 10 - 22 und ganz besonders bevorzugt 12 - 22 Kohlenstoffatomen. Einsetzbar im Sinne der Erfindung sind z.B. Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole.

Für Stiftformulierungen werden häufig Wachse verwendet. Als natürliche oder synthetische Wachse können erfindungsgemäß eingesetzt werden feste Paraffine oder Isoparaffine, Pflanzenwachse wie Candelillawachs, Carnaubawachs, Espartograswachs, Japanwachs, Korkwachs, Zuckerrohrwachs, Ouricurywachs, Montanwachs, Sonnenblumenwachs, Fruchtwachse und tierische Wachse, wie z. B. Bienenwachse und andere Insektenwachse, Walrat, Schellackwachs, Wollwachs und Bürzelfett, weiterhin Mineralwachse, wie z. B. Ceresin und Ozokerit oder die petrochemischen Wachse, wie z. B. Petrolatum, Paraffinwachse, Microwachse aus Polyethylen oder Polypropylen und Polyethylenglycolwachse. Es kann vorteilhaft sein, hydrierte oder gehärtete Wachse einzusetzen. Weiterhin sind auch chemisch modifizierte Wachse, insbesondere die Hartwachse, z. B. Montanesterwachse, Sasolwachse und hydrierte Jojobawachse, einsetzbar.

Weiterhin geeignet sind die Triglyceride gesättigter und gegebenenfalls hydroxylierter C₁₆₋₃₀-Fettsäuren, wie z. B. gehärtete Triglyceridfette (hydriertes Palmöl, hydriertes Kokosöl, hydriertes Rizinusöl), Glyceryltribehenat oder Glyceryltri-12-hydroxystearat, weiterhin synthetische Vollester aus Fettsäuren und Glykolen (z. B. Syncrowachs®) oder Polyolen mit 2 - 6 C-Atomen, Fettsäuremonoalkanolamide mit einem C₁₂₋₂₂-Acylrest und einem C₂₋₄-Alkanolrest, Ester aus gesättigten und/oder ungesättigten, verzweigten und/oder unverzweigten Alkancarbonsäuren einer Kettenlänge von 1 bis 80 C-Atomen und gesättigten und/oder ungesättigten, verzweigten und/oder unverzweigten Alkoholen einer Kettenlänge von 1 bis 80 C-Atomen, darunter z. B. synthetische Fettsäure-Fettalkoholester wie Stearylstearat oder Cetylpalmitat, Ester aus aromatischen Carbonsäuren, Dicarbonsäuren bzw. Hydroxycarbonsäuren (z. B. 12-Hydroxystearinsäure) und gesättigten und/ oder ungesättigten, verzweigten und/ oder unverzweigten Alkoholen einer Kettenlänge von 1 bis 80 C-Atomen, Lactide langkettiger Hydroxycarbonsäuren und Vollester aus Fettalkoholen und Di- und Tricarbonsäuren, z. B. Dicetylsuccinat oder Dicetyl-/stearyladipat, sowie Mischungen dieser Substanzen, sofern die einzelnen Wachskomponenten oder ihre Mischung bei Raumtemperatur fest sind.
Besonders bevorzugt ist, die Wachskomponenten zu wählen aus der Gruppe der Ester aus gesättigten, unverzweigten Alkancarbonsäuren einer Kettenlänge von 14 bis 44 C-Atomen und gesättigten, unverzweigten Alkoholen einer Kettenlänge von 14 bis 44 C-Atomen, sofern die Wachskomponente oder die Gesamtheit der Wachskomponenten bei Raumtemperatur fest sind. Insbesondere vorteilhaft können die Wachskomponenten aus der Gruppe der C₁₆₋₃₆-Alkylstearate, der C₁₀₋₄₀-Alkylstearate, der C₂₋₄₀-Alkylisostearate, der C₂₀₋₄₀-Dialkylester von Dimersäuren, der C₁₈₋₃₈-Alkylhydroxystearoylstearate, der C₂₀₋₄₀-Alkylerucate gewählt werden, ferner sind C₃₀₋₅₀-Alkylbienenwachs sowie Cetearylbehenat einsetzbar. Auch Silikonwachse, zum Beispiel Stearyltrimethylsilan/Stearylalkohol sind gegebenenfalls vorteilhaft. Besonders bevorzugte Wachskomponenten sind die Ester aus gesättigten, einwertigen C₂₀-C₆₀-Alkoholen und gesättigten C₈-C₃₀-Monocarbonsäuren, insbesondere ein C₂₀-C₄₀-Alkylstearat bevorzugt, das unter dem Namen Kesterwachs® K82H von der Firma Koster Keunen Inc. erhältlich ist. Das Wachs oder die Wachskomponenten sollten bei 25° C fest sein, jedoch im Bereich von 35 - 95°C schmelzen, wobei ein Bereich von 45 - 85 °C bevorzugt ist.
Natürliche, chemisch modifizierte und synthetische Wachse können alleine oder in Kombination eingesetzt werden.

Die Wachskomponenten sind in einer Menge von 0,1 bis 40 Gew.-%, bezogen auf die Gesamtzusammensetzung, vorzugsweise 0,5 bis 30 Gew.-% und insbesondere 1 - 15 Gew.-% enthalten.

Die erfindungsgemäßen Zusammensetzungen können weiterhin wenigstens ein unpolares oder polares flüssiges Öl, das natürlich oder synthetisch sein kann, enthalten.
Die polare Ölkomponente kann ausgewählt sein aus pflanzlichen Ölen, z. B. Sonnenblumenöl, Olivenöl, Sojaöl, Rapsöl, Mandelöl, Jojobaöl und den flüssigen Anteilen des Kokosöls sowie synthetischen Triglyceridölen, aus Esterölen, das heißt den Estern von C₆₋₃₀-Fettsäuren mit C₂₋₃₀-Fettalkoholen, aus Dicarbonsäureestern wie Di-n-butyladipat, Di-(2-ethylhexyl)-adipat und Di-(2-ethylhexyl)-succinat sowie Diolestern wie Ethylenglykoldioleat und Propylenglykoldi(2-ethylhexanoat), aus symmetrischen, unsymmetrischen oder cyclischen Estern der Kohlensäure mit Fettalkoholen, beispielsweise beschrieben in der DE-OS 197 56 454, Glycerincarbonat oder Dicaprylylcarbonat (Cetiol® CC), aus Mono-, Di- und Trifettsäureestern von gesättigten und/oder ungesättigten linearen und/oder verzweigten Fettsäuren mit Glycerin, z. B. Cutina® MD, aus verzweigten Alkanolen, z. B. Guerbet-Alkoholen mit einer einzigen Verzweigung am Kohlenstoffatom 2 wie 2-Hexyldecanol, 2-Octyldodecanol, Isotridecanol und Isohexadecanol, aus Alkandiolen, z. B. den aus Epoxyalkanen mit 12 - 24 C-Atomen durch Ringöffnung mit Wasser erhältlichen vicinalen Diolen, aus Etheralkoholen, z. B. den Monoalkylethern des Glycerins, des Ethylenglycols, des 1,2-Propylenglycols oder des 1,2-Butandiols, aus Dialkylethern mit jeweils 12 - 24 C-Atomen, z. B. den Alkyl-methylethern oder Di-n-alkylethern mit jeweils insgesamt 12 - 24 C-Atomen, insbesondere Di-n-octylether (Cetiol®OE ex Cognis), sowie aus Anlagerungsprodukten von Ethylenoxid und/oder Propylenoxid an ein- oder mehrwertige C₃₋₂₀-Alkanole wie Butanol und Glycerin, z. B. PPG-3-Myristylether (Witconol® APM), PPG-14-Butylether (Ucon Fluid® AP), PPG-15-Stearylether (Arlamol® E), PPG-9-Butylether (Breox® B25) und PPG-10-Butandiol (Macol® 57).
Die unpolare Ölkomponente kann ausgewählt sein aus flüssigen Paraffinölen, Isoparaffinölen, z. B. Isohexadecan und Isoeicosan, aus synthetischen Kohlenwasserstoffen, z. B. 1,3-Di-(2-ethyl-hexyl)-cyclohexan (Cetiol® S), sowie aus flüchtigen und nichtflüchtigen Siliconölen, die cyclisch, wie z. B. Decamethylcyclopentasiloxan und Dodecamethylcyclohexasiloxan, oder linear sein können, z. B. Polydialkylsiloxane, wie bevorzugt Hexamethyldisiloxan (L₂), Octamethyltrisiloxan (L₃), 3-Hexylheptamethyltrisiloxan (z. B. DC 2-1731 von Dow Corning), Decamethyltetrasiloxan (L₄), Dodecamethylpentasiloxan (L₅), Polyalkylarylsiloxane, z. B. Poly(methylphenylsiloxan), sowie Mischungen der vorgenannten Substanzen, insbesondere bevorzugt Mischungen aus L₃ und L₄, wie sie unter der Bezeichnung DC 2-1184 von Dow Corning erhältlich sind, weiterhin bevorzugt die unter der Bezeichnung Dow Corning® 200 Fluid erhältlichen Polymere mit unterschiedlicher Kettenlänge und Viskosität (INCI-Bezeichnung: Dimethicone), insbesondere mit Viskositäten von 0,65 cSt, 1,0 cSt, 1,5 cSt, 2,0 cSt, 5,0 cSt, 6,0 cSt, 10 cSt, 100 cSt und höhere Viskositäten. Weitere bevorzugte Siliconöle sind im Handel erhältlich z. B. unter der Bezeichnung Dow Corning® 190, 200, 244, 245, 344 oder 345 und Baysilon® 350 M.

Die erfindungsgemäßen Zusammensetzungen können weiterhin wenigstens einen wasserlöslichen Alkohol enthalten. Unter Wasserlöslichkeit versteht man erfindungsgemäß, dass sich wenigstens 5 Gew.-% des Alkohols bei 20 °C klar lösen oder aber - im Falle langkettiger oder polymerer Alkohole - durch Erwärmen der Lösung auf 50 °C bis 60 °C in Lösung gebracht werden können. Geeignet sind je nach Darreichungsform einwertige Alkohole wie z. B. Ethanol, Propanol oder Isopropanol. Weiterhin geeignet sind wasserlösliche Polyole. Hierzu zählen wasserlösliche Diole, Triole und höherwertige Alkohole sowie Polyethylenglycole. Unter den Diolen eignen sich C₂-C₁₂-Diole, insbesondere 1,2-Propylenglycol, Butylenglycole wie z. B. 1,2-Butylenglycol, 1,3-Butylenglycol und 1,4-Butylenglycol, Hexandiole wie z. B. 1,6-Hexandiol. Weiterhin bevorzugt geeignet sind Glycerin und insbesondere Diglycerin und Triglycerin, 1,2,6-Hexantriol sowie die Polyethylenglycole (PEG) PEG-400, PEG-600, PEG-1000, PEG-1550, PEG-3000 und PEG-4000.
Die Menge des Alkohols oder des Alkohol-Gemisches in den erfindungsgemäßen Zusammensetzungen beträgt 1 - 50 Gew.-% und vorzugsweise 5 - 40 Gew.-%, bezogen auf die gesamte Zusammensetzung. Erfindungsgemäß kann sowohl ein Alkohol als auch ein Gemisch mehrerer Alkohole eingesetzt werden.

Die erfindungsgemäßen Zusammensetzungen können im wesentlichen wasserfrei sein, das heißt maximal 5 Gew.-%, bevorzugt maximal 1 Gew.-% Wasser, bezogen auf das Gewicht der gesamten kosmetischen Zusammensetzung, enthalten.

In wasserhaltigen Darreichungsformen beträgt der Wassergehalt 5 - 98 Gew.-%, bevorzugt 10 - 90 und besonders bevorzugt 15 - 85 Gew.-%, bezogen auf die gesamte kosmetische Zusammensetzung.

Die erfindungsgemäßen Zusammensetzungen können weiterhin wenigstens ein hydrophil modifiziertes Silicon enthalten. Sie ermöglichen die Formulierung hochtransparenter Zusammensetzungen, reduzieren die Klebrigkeit und hinterlassen ein frisches Hautgefühl. Unter hydrophil modifizierten Siliconen werden erfindungsgemäß Polyorganosiloxane mit hydrophilen Substituenten verstanden, welche die Wasserlöslichkeit der Silicone bedingen. Erfindungsgemäß wird unter Wasserlöslichkeit verstanden, dass sich wenigstens 2 Gew.-% des mit hydrophilen Gruppen modifizierten Silicons in Wasser bei 20 °C lösen. Entsprechende hydrophile Substituenten sind beispielsweise Hydroxy-, Polyethylenglycol- oder Polyethylenglycol/Polypropylenglycol-Seitenketten sowie ethoxylierte Ester-Seitenketten. Erfindungsgemäß bevorzugt geeignet sind hydrophil modifizierte Silicon-Copolyole, insbesondere solche Verbindungen mit der früheren INCI-Bezeichnung Dimethicone Copolyol, die beispielsweise von Wacker-Chemie unter der Bezeichnung Belsil® DMC 6031, Belsil® DMC 6032, Belsil® DMC 6038 oder Belsil® DMC 3071 VP bzw. von Dow Corning unter der Bezeichnung DC 2501 bzw. von Degussa unter der Bezeichnung Abil EM 97 im Handel sind. Besonders bevorzugt geeignet ist die Verwendung von Belsil® DMC 6038 bzw. Abil EM 97, da es die Formulierung hochtransparenter Zusammensetzungen ermöglicht, die beim Verbraucher eine höhere Akzeptanz erreichen. Erfindungsgemäß kann auch ein beliebiges Gemisch dieser Silicone eingesetzt werden.
Weitere hydrophil modifizierte Silicon-Copolyole sind ausgewählt aus den Poly-(C₂-C₃)-alkylen-modifizierten Siliconen, deren frühere INCI-Bezeichnung Dimethicone Copolyol lautete, mit den aktuellen INCI-Bezeichnungen PEG-x Dimethicone (mit x = 2 - 20, bevorzugt 3 - 17, besonders bevorzugt 11 - 12), Bis-PEG-y Dimethicone (mit y = 3 - 25, bevorzugt 4 - 20), PEG/PPG a/b Dimethicone (wobei a und b unabhängig voneinander für Zahlen von 2 - 30, bevorzugt 3 - 30 und besonders bevorzugt 12 - 20, insbesondere 14 - 18, stehen), Bis-PEG/PPG-c/d Dimethicone (wobei c und d unabhängig voneinander für Zahlen von 10 - 25, bevorzugt 14 - 20 und besonders bevorzugt 14 - 16, stehen) und Bis-PEG/PPG-e/f PEG/PPG g/h Dimethicone (wobei e, f, g und h unabhängig voneinander für Zahlen von 10 - 20, bevorzugt 14 - 18 und besonders bevorzugt 16, stehen). Bevorzugt sind PEG/PPG-18/18 Dimethicone, das in einer 1:9-Mischung mit Cyclomethicone unter der Bezeichnung DC 3225 C bzw. DC 5225 C von Dow Corning im Handel erhältlich ist, sowie Bis-PEG/PPG-14/14 Dimethicone, das in einer Mischung mit Cyclomethicone unter der Bezeichnung Abil EM 97 (Goldschmidt) im Handel erhältlich ist.
Weiterhin bevorzugt sind Poly-(C₂-C₃)alkylen-modifizierte Silicone, die mit C₄-C₁₈-Alkylgruppen hydrophob modifiziert sind, besonders bevorzugt Cetyl PEG/PPG-10/1 Dimethicone (früher: Cetyl Dimethicone Copolyol, erhältlich unter der Bezeichnung Abil EM 90), weiterhin Alkyl Methicone Copolyole und Alkyl Dimethicone Ethoxy Glucoside.

Die Menge des hydrophil modifizierten Silicons oder des Alkohol-Gemisches in den erfindungsgemäßen Zusammensetzungen beträgt 0,1 - 10 Gew.-%, bevorzugt 0,5 - 8 Gew.-% und besonders bevorzugt 1 - 6 Gew.-%, bezogen auf das Gesamtgewicht der Zusammensetzung.

Die erfindungsgemäßen Zusammensetzungen können weiterhin wenigstens ein wasserlösliches Tensid enthalten. Als wasserlösliche Tenside eignen sich grundsätzlich alle in Wasser bei 20°C zu mindestens 1 Gew.-% löslichen Tenside. Obwohl die Struktur und lonogenität an sich unerheblich sind, scheinen nichtionische Tenside, insbesondere die bei Normaltemperatur (20°C) festen Anlagerungsprodukte des Ethylenoxids an Fettstoffmoleküle mit wenigstens einer alkoxylierbaren Gruppe, bevorzugt geeignet zu sein. Solche geeigneten Tenside sind z.B. die Anlagerungsprodukte von 5 - 50 Mol Ethylenoxid an lineare Fettalkohole mit 16 - 22 C-Atomen, an Fettsäuren mit 5 - 22 C-Atomen, an Fettsäurealkanolamide, an Fettsäuremonoglyceride, an Sorbitan-Fettsäuremonoester, an Fettsäurealkanolamide, an Fettsäureglyceride, z.B. an gehärtetes Rizinusöl, an Methylglucosidmonofettsäureester und Gemische davon.

Die erfindungsgemäßen Zusammensetzungen enthalten in einer bevorzugten Ausführungsform mindestens einen Antitranspirant-Wirkstoff. Als Antitranspirant-Wirkstoffe eignen sich wasserlösliche adstringierende metallische Salze, insbesondere anorganische und organische Salze des Aluminiums, Zirkoniums und Zinks bzw. beliebige Mischungen dieser Salze. Erfindungsgemäß wird unter Wasserlöslichkeit eine Löslichkeit von wenigstens 5 g Aktivsubstanz pro 100 g Lösung bei 20 °C verstanden. Erfindungsgemäß verwendbar sind beispielsweise Alaun (KAl(SO₄)₂ · 12 H₂O), Aluminiumsulfat, Aluminiumlactat, Natrium-Aluminium-Chlorhydroxylactat, Aluminiumchlorhydroxyallantoinat, Aluminiumchlorohydrat, Aluminiumsulfocarbolat, Aluminium-Zirkonium-Chlorohydrat, Zinkchlorid, Zinksulfocarbolat, Zinksulfat, Zirkoniumchlorohydrat und Aluminium-Zirkonium-Chlorohydrat-Glycin-Komplexe. Bevorzugt enthalten die Zusammensetzungen ein adstringierendes Aluminiumsalz, insbesondere Aluminiumchlorohydrat, und/oder eine Aluminium-Zirkonium-Verbindung. Die Antitranspirant-Wirkstoffe werden bei wässrigen Applikationen als wässrige Lösungen eingesetzt. In wasserfreien Zusammensetzungen werden die Antitranspirant-Wirkstoffe in fester Form eingesetzt. Sie sind in den erfindungsgemäßen Zusammensetzungen in einer Menge von 1 - 40 Gew.-%, vorzugsweise 5 - 30 Gew.-% und insbesondere 10 - 25 Gew.-% enthalten (bezogen auf die Menge der Aktivsubstanz in der Gesamtzusammensetzung bzw. bei aerosolförmigen Produkten bezogen auf das treibmittelfreie Konzentrat der Zusammensetzung). Aluminiumchlorohydrate werden beispielsweise pulverförmig als Micro Dry® Ultrafine von Reheis, als Chlorhydrol®, in aktivierter Form als Reach® 501 von Reheis sowie in Form einer wäßrigen Lösung als Locron® L von Clariant vertrieben. Unter der Bezeichnung Reach® 301 wird ein Aluminiumsesquichlorohydrat von Reheis angeboten. Auch die Verwendung von Aluminium-Zirkonium-Tetrachlorohydrex-Glycin-Komplexen, die beispielsweise von Reheis unter der Bezeichnung Rezal® 36G im Handel sind, ist erfindungsgemäß besonders vorteilhaft. Einsetzbar sind auch die oben genannten Antitranspirant-Wirkstoffe, die durch einen modifizierten Herstellprozeß eine erhöhte schweißreduzierende Wirkung besitzen.

Weiterhin können die erfindungsgemäßen Zusammensetzungen keimhemmende oder desodorierende Wirkstoffe enthalten. Geeignet sind insbesondere Organohalogenverbindungen sowie -halogenide, quartäre Ammoniumverbindungen, eine Reihe von Pflanzenextrakten und Zinkverbindungen. Bevorzugt sind Chlorhexidin und Chlorhexidingluconat, Benzalkoniumhalogenide und Cetylpyridiniumchlorid. Desweiteren sind Natriumbicarbonat, Natriumphenolsulfonat und Zinkphenolsulfonat sowie z. B. die Bestandteile des Lindenblütenöls einsetzbar.
Weitere antibakteriell wirksame Deodorans-Wirkstoffe sind Lantibiotika, Glycoglycerolipide, Sphingolipide (Ceramide), Sterine und andere Wirkstoffe, die die Bakterienadhäsion an der Haut inhibieren, z. B. Glycosidasen, Lipasen, Proteasen, Kohlenhydrate, Di- und Oligosaccharidfettsäureester.

Weiterhin können die erfindungsgemäß verwendeten Zusammensetzungen mit Enzyminhibitoren gegen Körpergeruch, z. B. Inhibitoren für Lipase, Arylsulfatase, β-Glucuronidase, Aminoacylase etc. kombiniert werden. Auch Kombinationen mit Antioxidantien sind möglich.

Weiterhin können die erfindungsgemäß verwendeten Zusammensetzungen mindestens eine den Haarwuchs inhibierende Substanz enthalten. Geeignete Substanzen, die den Haarwuchs inhibieren, sind insbesondere ausgewählt aus dem Rohstoff Pilinhib Veg von Laboratoires Sérobiologiques mit der INCI-Deklaration: Propylene glycol, Hydrolyzed Soy Protein, Hypericum Perforatum Extract, Hamamelis Virginiana Extract, Arnica Montana Flower Extract, Urea, Salix Alba Bark Extract, Menthol, Salicylic acid, weiterhin der Rohstoff ARP 100 von Greentech S.A./Rahn, der Extrakte aus den Beeren von Saw Palmetto (serenoa repens) enthält, weiterhin der Rohstoff Xyleine von Impag / Seporga mit der INCI-Deklaration: Xylitol and Citrus Medica Limonum (Lemon) Fruit Extract and Carica Papaya (Papaya) Fruit Extract and Olea europaea (olive) leaf extract, sowie der Rohstoff Capislow von Sederma, der Lecithinvesikel mit einem hydroglycolischen Extrakt aus Larrea tridendata enthält.

Flüssige und gelförmige Darreichungsformen können Verdickungsmittel enthalten, z. B. Celluloseether, wie Hydroxypropylcellulose, Hydroxyethylcellulose, hydrophob modifizierte Hydroxyethylcellulose und Methylhydroxypropylcellulose, verdickende Polymere auf Basis von Polyacrylaten, die gewünschtenfalls vernetzt sein können, z. B. die Carbopoltypen oder Pemulen®-Produkte, oder auf Basis von Polyacrylamiden oder sulfonsäuregruppenhaltigen Polyacrylaten, z. B Sepigel® 305 oder Simulgel® EG, weiterhin anorganische Verdicker, z. B. Bentonite und Hectorite (Laponite®).
Die erfindungsgemäßen Zusammensetzungen können weitere kosmetisch und dermatologisch wirksame Stoffe enthalten, wie beispielsweise entzündungshemmende Substanzen, Feststoffe, ausgewählt aus Kieselsäuren, z. B. Aerosil®-Typen, Kieselgelen, Siliciumdioxid, Tonen, z. B. Bentonite oder Kaolin, Magnesiumaluminiumsilikaten, z. B. Talkum, Bornitrid, Titandioxid, das gewünschtenfalls beschichtet sein kann, gegebenenfalls modifizierten Stärken und Stärkederivaten, Cellulosepulvern und Polymerpulvern, desweiteren Pflanzenextrakte, Proteinhydrolysate, Vitamine, Parfümöle, Sebostatika, Anti-Akne-Wirkstoffe sowie Keratolytika.

Die kosmetischen Deodorant- oder Antitranspirant-Zusammensetzungen, die die erfindungsgemäß verwendeten Inhibitoren für Gram-positive Kokken, insbesondere *Peptostreptococcus,* enthalten, können, soweit sie flüssig vorliegen, auf flexible und saugfähige Träger aufgebracht und als Deodorant- oder Antitranspirant-Tücher oder Schwämmchen angeboten werden. Als flexible und saugfähige Träger im Sinne der Erfindung eignen sich z. B. Träger aus Textilfasern, Kollagen oder polymeren Schaumstoffen. Als Textilfasern können sowohl Naturfasern wie Cellulose (Baumwolle, Leinen), Seide, Wolle, Regeneratcellulose (Viskose, Rayon), Cellulosederivate als auch synthetische Fasern wie z.B. Polyester, Polyacrylnitril, Polyamid- oder Polyolefinfasern oder Mischungen solcher Fasern gewebt oder ungewebt verwendet werden. Diese Fasern können zu saugfähigen Wattepads, Vliesstoffen oder zu Geweben oder Gewirken verarbeitet sein. Auch flexible und saugfähige polymere Schaumstoffe, z. B. Polyurethanschäume und Polyamidschäume sind geeignete Substrate. Das Substrat kann eine, zwei, drei sowie mehr als drei Lagen aufweisen, wobei die einzelnen Lagen aus gleichen oder unterschiedlichen Materialien bestehen können. Jede Substratschicht kann eine homogene oder eine inhomogene Struktur mit beispielsweise verschiedenen Zonen unterschiedlicher Dichte aufweisen.

Als saugfähig im Sinne der Erfindung sind solche Trägersubstrate anzusehen, die bei 20° C wenigstens 10 Gew.-%, bezogen auf das Trockengewicht, an Wasser adsorptiv bzw. kapillar binden können. Bevorzugt eignen sich aber solche Träger, die wenigstens 100 Gew.-% Wasser adsorptiv und kapillar binden können.

Die Ausrüstung der Trägersubstrate erfolgt in der Weise, dass man die saugfähigen, flexiblen Trägersubstrate, bevorzugt aus Textilfasern, Kollagen oder polymeren Schaumstoffen mit den erfindungsgemäßen Zusammensetzungen behandelt bzw. ausrüstet und gegebenenfalls trocknet. Dabei kann die Behandlung (Ausrüstung) der Trägersubstrate nach beliebigen Verfahren, z. B. durch Aufsprühen, Tauchen und Abquetschen, Durchtränken oder einfach durch Einspritzen der erfindungsgemäßen Zusammensetzung in die Trägersubstrate erfolgen.

Erfindungsgemäß bevorzugt ist weiterhin die Darreichungsform als Aerosol, wobei die kosmetische Zusammensetzung ein Treibmittel, ausgewählt aus Propan, Butan, Isobutan, Pentan, Isopentan, Dimethylether, Fluorkohlenwasserstoffen und Fluorchlorkohlenwasserstoffen sowie Mischungen hiervon enthält.

In besonders bevorzugten erfindungsgemäßen Verfahren zur Verringerung von Körpergeruch wird/werden die an der Körpergeruchsbildung beteiligten Gram-positiven Kokken, insbesondere Bakterien der Gattung *Peptostreptococcus* inhibierende(n) Substanz(en) hinsichtlich ihrer Menge und/oder ihrer Art geschlechtsspezifisch eingesetzt. Hierbei sind insbesondere bevorzugte Verfahren dadurch gekennzeichnet, dass die an der Körpergeruchsbildung beteiligte Gram-positive anaerobe Kokken, insbesondere Bakterien der Gattung *Peptostreptococcus* inhibierende(n) Substanz(en) zur Verringerung von Körpergeruch beim Mann eingesetzt wird/werden.

Die nachfolgenden Beispiele sollen den Gegenstand der Erfindung verdeutlichen, ohne ihn hierauf zu beschränken.

### Durchführung der Inhibitionsversuche

Eine LB-Vorkultur von Anaerococcus octavius (Peptostreptococcus octavius) wurde mit Medium auf eine Keimdichte von 10⁴ cfu/ml eingestellt und in 100 ml-Aliquots auf Erlenmeyer-Kolben verteilt. Dazu wurden die zu testenden Deodorant-Formulierungen gegeben, wobei die relevanten Bestandteile wie folgt konzentriert waren (Endkonzentration im Nährmedium):

| | |
|---|---|
| Sensiva | 0,4 Gew.-% |
| Phenoxyethanol | 0,8 Gew.-% |
| Protectate MOD 2 | 0,2 Gew.-% |

Die Kultur wurde dann mit 100 Upm bei 37°C inkubiert. Nach 24 h erfolgte eine Keimzahlbestimmung über Ausplattieren im Vergleich zu einer unbehandelten Kontrolle.

**Tabelle 1**

| Nachweis der Inhibierung von Anaerococcus octavius durch eine erfindungsgemäße Deodorant-Formulierung | | |
|---|---|---|
| Zeit [h] | Kontrolle (unbehandelt) | Deoformulierung |
| 0 | 1,23 E+05 | 1,25 E+05 |
| 4 | 1,91 E+05 | 9,69 E+03 |
| 8 | 2,18 E+06 | 5,33 E+02 |
| 24 | 1,76 E+08 | 2,73 E+02 |

Während sich die Keimzahl in der unbehandelten Kontrollkultur innerhalb des Testzeitraums von 24 Stunden von 1,23 E+05 auf 1,76 E+08 erhöhte, nahm sie in der mit einer erfindungsgemäßen Deodorant-Formulierung behandelten Kultur von 1,25 E+05 auf 2,73 E+02 ab.

Weiterhin wurden die Wirkstoffe mit Hilfe des Filterpapier/Oberflächenverfahrens vermessen. Der Testkeim Anaerococcus octavius (Peptostreptococcus octavius) war erhältlich als DSM 10024; er wurde 5 Tage bei 37 °C vorinkubiert. 0,3 ml Wirkstofflösung bzw. 0,3 g wirkstoffhaltiges Produkt wurden auf eine Filterpapierscheibe mit einem Durchmesser von 4,5 cm aufgebracht, anschließend wurde die Filterpapierscheibe auf den bakterienhaltigen Agar gelegt. Nach der Inkubationszeit wurde die Größe des gebildeten Hemmhofes vermessen. Bei den Lösungen, für die kein Hemmhof detektiert werden konnte, weil der Wirkstoff nicht in den Agar penetriert war, wurde zusätzlich das Wachstum unter den Filterpapierscheiben beurteilt.
Es wurden folgende Inhibitoren identifiziert:

| Inhibitor | Gew.-% Aktivsubstanz | Medium | pH-Wert tel quel | pH-Wert nach Einstellung | Durchmesser des Hemmhofes [mm] | Wachstum unter der Filterscheibe |
|---|---|---|---|---|---|---|
| Arlatone DCA (= Octadecenedioic acid) | 2 | Ethanol | - | - | 4 | kein Wachstum |
| Wasserkontrolle | | Wasser | - | | 0 | nicht gehemmt |
| Aluminium chlorohydrate (50 Gew.-%ige wässrige Lösung von Reheis) | 8 | Wasser/ Ethanol (30 Vol.-% / 70 Vol.-%) | - | 4,2 | 0 | deutlich gehemmt |
| Aluminium chlorohydrate (50 Gew.-%ige wässrige Lösung von Reheis) | 20 | Wasser | - | 4,2 | 0 | leicht gehemmt |
| REACH AZP 908 (Powder) von Reheis = Aluminium Zirconium Tetrachlorohydrex Gly | 22 | Wasser | | ca. 4 | 0 | kein Wachstum |
| Rezal 36 GP (Powder) von Reheis = Aluminium Zirconium Tetrachlorohydrex Gly | 22 | Wasser | | ca. 4 | 0 | kein Wachstum |
| Rezal 36 GP conc. Solution (46 Gew.-%ig in Wasser) von Reheis = Aluminium Zirconium Tetrachlorohydrex Gly | 22 | Wasser | | ca. 4 | 0 | kein Wachstum |
| Zinkgluconat | 0,5 | Wasser | 6,5 | nach Zugabe von Citronensäure: 5,1 | 0 | deutlich gehemmt |
| Plantaren 2000 (50 Gew.-%ige wässrige Zubereitung) = Decyl Glucoside | 1 | Wasser | 10,8 | nach Zugabe von Citronensäure: 6,0 | 3 - 4 | kein Wachstum |
| Sensiva SC 50 = 2-Ethylhexylglycerinether | 0,5 | Wasser/ Ethanol (50 Vol.-% / 50 Vol.-%) | 6,65 | nach Zugabe von Citronensäure: 5,1 | 0 | deutlich gehemmt |
| Protectate HR | 0,8 | Ethanol | - | - | 0 | deutlich gehemmt |
| Wasserkontrolle | - | Wasser | - | - | 0 | nicht gehemmt |

Die folgenden Formulierungsbeispiele sollen den Gegenstand der Erfindung verdeutlichen, ohne ihn hierauf zu beschränken.

### 2. Deodorant-Aerosolsprays

| | **1** | **2** | **3** | **4** | **5** |
|---|---|---|---|---|---|
| Triethylcitrate | 1,00 | 5,00 | 6,00 | 1,50 | 3,00 |
| 2-Ethylhexylglycerin | 0,50 | 0,10 | 0,50 | 0,20 | 0,30 |
| Phenoxyethanol | 0,30 | 0,50 | 0,10 | 0,40 | 0,60 |
| Parfum | 0,50 | 1,50 | 1,00 | 1,00 | 1,00 |
| Plant Extract | 0,05 | - | 0,20 | - | 0,00 |
| Aroma | 0,50 | 0,01 | 0,10 | 0,10 | 0,05 |
| Vitamin E-Acetate | - | - | - | 0,05 | 0,10 |
| Bisabolol | - | - | 0,10 | 0,10 | - |
| Protectate MOD 2 | 0,40 | - | 0,20 | 0,10 | 0,20 |
| Protectate HR | - | - | - | - | - |
| Arlatone Dioic Acid | - | 1,00 | - | - | - |
| Hydrocarbon Propellant | 85,00 | 60,00 | 70,00 | 75,00 | 70,00 |
| Alcohol Denat. | ad 100 | ad 100 | ad 100 | ad 100 | ad 100 |

### 3. Deodorant-Aerosolsprays

| | **1** | **2** | **3** | **4** |
|---|---|---|---|---|
| Isopropylmyristate | 1,00 | 10,00 | 5,00 | 2,00 |
| Phenoxyethanol | 0,30 | 0,50 | 0,10 | 0,20 |
| Parfum | 0,50 | 1,50 | 1,00 | 0,50 |
| Plant Extract | 0,05 | - | 0,20 | 0,50 |
| 2-Ethylhexylglycerin | 0,40 | 0,10 | 1,50 | 0,80 |
| Protectate MOD 2 | 0,20 | 0,30 | - | - |
| Protectate HR | - | - | 0,10 | 0,20 |
| Arlatone Dioic Acid | - | - | 0,50 | - |
| Hydrocarbon Propellant | 75,00 | 85,00 | 78,00 | 60,00 |
| Alcohol Denat. | ad 100 | ad 100 | ad 100 | ad 100 |

### 4. Antitranspirant-Aerosolsprays

| | **1** | **2** | **3** | **4** |
|---|---|---|---|---|
| Aluminum chlorohydrate | 4,00 | 10,00 | | |
| Aluminum chlorohydrate activated | | | 2,00 | 10,00 |
| Disteardimonium Hectorite / Propylene Carbonate | 0,50 | 1,50 | 0,80 | 1,20 |
| Parfum | 0,80 | 0,50 | 1,00 | 1,50 |
| Plant Extract | 0,05 | 0,50 | 0,10 | 0,20 |
| Encapsulated Parfum / Active (Fircaps*) | 1,50 | 0,10 | 1,50 | 0,10 |
| Protectate MOD 2 | 0,05 | 0,15 | | |
| Protectate HR | | | 0,20 | 0,10 |
| Zinkgluconate | | | 0,05 | |
| Hydrocarbon Propellant | 85,00 | 75,00 | 80,00 | 60,00 |
| Cyclopentasiloxane/ Cylclohexasiloxane | ad 100 | ad 100 | ad 100 | ad 100 |

| | | | | |
|---|---|---|---|---|
| * Fircaps = in Cellulose-Derivaten verkapselte Parfüm-Menthyllactat-Mischung oder allgemein in Cellulose-Derivaten verkapselte Parfüm-Cooling Agent-Mischung, erhältlich von Firmenich | | | | |

### 5. Antitranspirant-Aerosolsprays

| | **1** | **2** | **3** | **4** |
|---|---|---|---|---|
| Aluminum chlorohydrate | 4,00 | 10,00 | - | - |
| Aluminum chlorohydrate activated | - | - | 2,00 | 10,00 |
| Disteardimonium Hectorite / Propylene Carbonate | 0,50 | 1,50 | 0,80 | 1,20 |
| Parfum | 0,80 | 0,50 | 1,00 | 1,50 |
| Plant Extract | 0,05 | 0,50 | 0,10 | 0,20 |
| Aroma | 0,50 | 0,01 | 0,05 | 0,10 |
| Di-C12-13 Alkyl Malate | - | 0,50 | 0,50 | 10,00 |
| Ethylhexylpalmitat | ad 100 | 5,00 | - | - |
| Protectate MOD 2 | 0,05 | 0,15 | - | - |
| Protectate HR | - | - | 0,20 | 0,10 |
| Zinkgluconate | - | - | 0,05 | - |
| Hydrocarbon Propellant | 85,00 | 75,00 | 80,00 | 60,00 |
| Cyclopentasiloxane/ Cylclohexasiloxane | - | ad 100 | ad 100 | ad 100 |

### 6. alkoholische Roll-on-Formulierungen

| | **1** | **2** | **3** | **4** | **5** |
|---|---|---|---|---|---|
| Alcohol Denat. | 35,00 | 30,00 | 28,00 | 30,00 | 40,00 |
| Aluminum Chlorhydrate 50% solution | 16,00 | 40,00 | 16,00 | 16,00 | - |
| Aluminum Zirconium Pentachlorohydrate 40% solution | - | - | - | - | 45,00 |
| Ceteareth-12 | 2,50 | 1,50 | 2,00 | 2,00 | 2,50 |
| Ceteareth-30 | 2,50 | 2,00 | 1,50 | 2,00 | 2,50 |
| Parfum | 0,70 | 1,00 | 1,50 | 1,20 | 1,20 |
| Tocopheryl Acetate | 0,05 | 0,10 | | 0,25 | 0,05 |
| Hydroxyethylcellulose | 0,50 | 0,30 | 0,40 | 0,60 | 0,50 |
| Zinc Gluconate | - | 0,10 | - | - | 0,10 |
| Plant Extract Colours approved for Cosmetics | - | - | 0,20 | 0,50 | 0,20 |
| | 0,0005 | 0,0010 | 0,0005 | 0,0100 | 0,0001 |
| 2-Ethylhexylglycerin | - | - | 0,40 | - | - |
| Protectate MOD 2 | 0,30 | 0,50 | - | - | - |
| Protectate HR | - | - | 0,20 | 0,60 | - |
| Aqua | ad 100 | ad 100 | ad 100 | ad 100 | ad 100 |

### 7. Roll-on-Emulsionen

| | **1** | **2** | **3** | **4** | **5** | **6** |
|---|---|---|---|---|---|---|
| Steareth-2 | 2,50 | 3,00 | 3,00 | 2,80 | 2,80 | 2,80 |
| PPG-15 Stearyl Ether | 2,00 | 3,00 | 2,00 | 2,20 | 2,00 | 2,00 |
| Steareth-21 | 1,00 | 1,00 | 3,00 | 1,00 | 1,30 | 1,30 |
| Aluminium Chlorohydrate 50% solution | 40,00 | 40,00 | 40,00 | 40,00 | 40,00 | - |
| Aluminium Zirconium Tetrachlorohydrex Gly, 35% solution | - | - | - | - | - | 63,00 |
| Allantoin | 0,10 | - | 0,10 | - | 0,10 | - |
| Plant Extract | - | 0,50 | 0,20 | - | - | 0,20 |
| Tocopheryl acetate | 0,05 | 0,05 | 0,05 | 0,25 | 0,25 | 0,25 |
| Parfum | 1,00 | 1,50 | 1,30 | 0,80 | 1,00 | 1,20 |
| 2-Ethylhexylglycerin | 0,30 | - | - | - | - | - |
| Protectate MOD 2 | 0,30 | 0,50 | - | - | - | - |
| Protectate HR | - | - | 0,20 | 0,60 | - | - |
| Arlatone Dioic Acid | - | - | - | - - | 1,00 | - |
| Aqua | ad 100 | ad 100 | ad 100 | ad 100 | ad 100 | ad 100 |

### 8. alkoholische Deodorant-Stifte

| | **1** | **2** | **3** | **4** |
|---|---|---|---|---|
| Alcohol Denat. | 40,00 | 40,00 | 35,00 | 30,00 |
| Propylene Glycol 1,2 | 30,32 | 32,32 | 32,32 | 38,00 |
| Butylene Glycol 1,3 | 12,00 | 10,00 | 15,00 | 12,00 |
| Sodium Palmitate | 3,10 | 3,50 | 2,80 | 3,10 |
| Sodium Stearate | 3,10 | 3,50 | 2,80 | 3,10 |
| Glycerin 86% | 2,00 | 1,00 | 1,70 | - |
| PPG-5-Laureth-5 | 0,50 | 1,00 | 1,00 | 0,50 |
| (Sodium Hydroxide 50%) | (2) | (2) | (2) | (2) |
| Parfum | 1,00 | 0,60 | 1,30 | 1,00 |
| Octyldodecanol | 1,00 | 0,50 | 1,00 | 0,70 |
| Phenoxyethanol | 1,00 | 0,50 | 1,00 | 0,50 |
| 2-Ethylhexylglycerin | 0,50 | - | 0,30 | - |
| Tocopheryl acetate | 0,05 | - | 0,10 | 0,25 |
| Plant Extract | 0,20 | - | 0,20 | 0,50 |
| PEG-40 Hydrogenated Castor Oil | 0,02 | - | - | 0,10 |
| Protectate MOD 2 | 0,30 | 0,50 | - | - |
| Protectate HR | - | - | 0,20 | 0,60 |
| Aqua | ad 100 | ad 100 | ad 100 | ad 100 |

### 9. nicht-alkoholische Deodorant-Stifte

| | **1** | **2** | **3** | **4** |
|---|---|---|---|---|
| PEG-8 | 40,00 | 45,00 | 50,00 | 46,00 |
| Sodium Palmitate | 2,50 | 2,50 | 2,50 | 2,50 |
| Sodium Stearate | 2,50 | 2,50 | 2,50 | 2,50 |
| Butylene Glycol 1,3 | 5,00 | 2,00 | 3,00 | 4,00 |
| PEG-14 Dimethicone | 1,00 | 2,00 | 1,50 | 1,50 |
| (Sodium Hydroxide 50%) | (1,5) | (1,2) | (1,4) | (1,3) |
| Phenoxyethanol | 1,00 | 2,00 | 0,50 | 1,00 |
| Parfum | 1,00 | 1,20 | 0,80 | 1,00 |
| 2-Ethylhexylglycerin | 0,30 | - | 0,30 | - |
| Steareth-10 | 0,20 | - | 0,20 | 0,20 |
| Plant Extract | 0,20 | 0,50 | - | 0,30 |
| Protectate MOD 2 | 0,30 | 0,50 | - | - |
| Protectate HR | - | - | 0,20 | 0,60 |
| Aqua | ad 100 | ad 100 | ad 100 | ad 100 |

### 10. Antitranspirant-Stifte

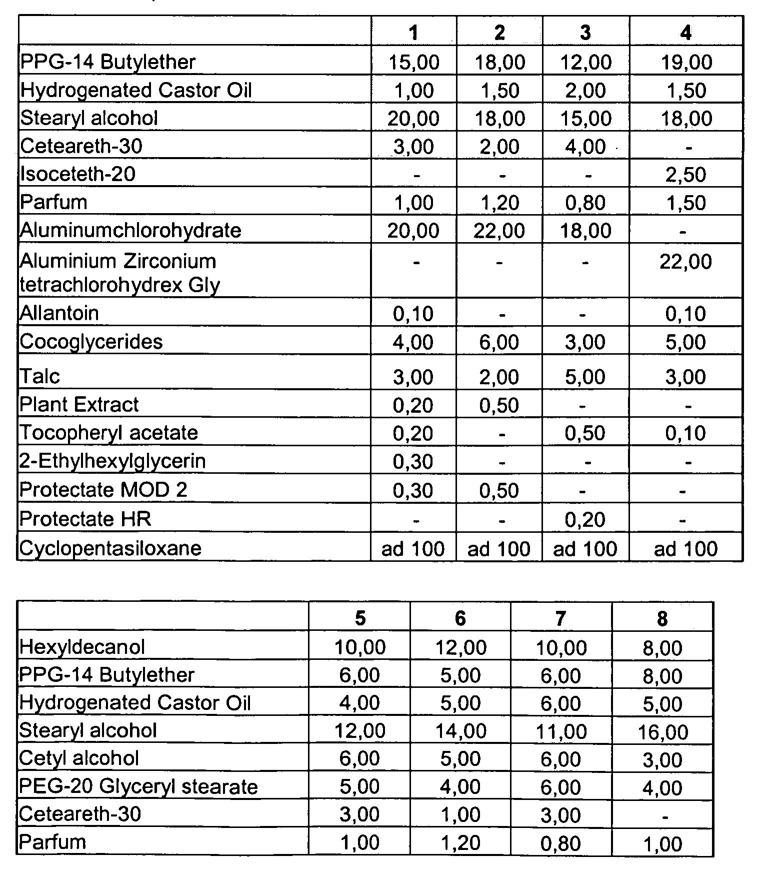

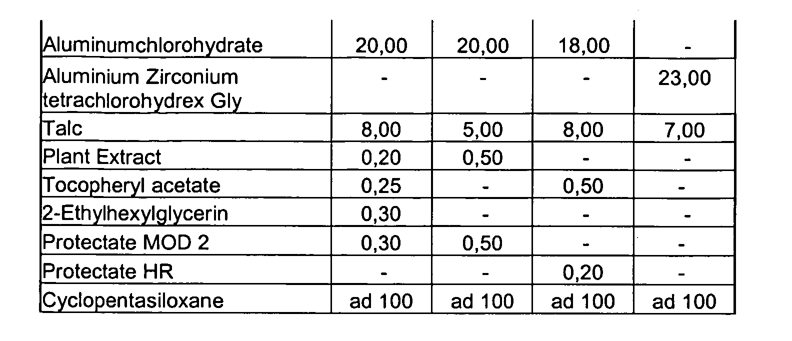

### 11. Deodorant-Sprays im Pumpdispenser (treibgasfrei)

| | **1** | **2** | **3** | **4** |
|---|---|---|---|---|
| Alcohol Denat. | 50,00 | 55,00 | 60,00 | 40,00 |
| Triethylcitrate | 2,50 | 3,50 | 4,00 | 3,00 |
| PEG-40 Hydrogenated Castor Oil | 1,00 | 0,50 | 0,50 | 2,00 |
| 2-Ethylhexylglycerin | 0,10 | 0,50 | - | - |
| Tocopheryl Acetate | 0,05 | 0,20 | 0,10 | - |
| Benzophenone-2 | 0,01 | 0,01 | 0,01 | 0,05 |
| Colours approved for Cosmetics | 0,0001 | 0,0005 | 0,0010 | - |
| Parfum | 0,80 | 1,00 | 2,00 | 1,50 |
| Protectate MOD 2 | 0,30 | 0,50 | - | - |
| Protectate HR | - | - | 0,20 | 0,60 |
| Phenoxyethanol | - | 1,00 | - | - |
| Aqua | ad 100 | ad 100 | ad 100 | ad 100 |

### 12. Antitranspirant-PIT-Emulsionen im Pumpdispenser (treibgasfrei)

| | **1** | **2** | **3** | **4** |
|---|---|---|---|---|
| Aluminum chlorohydrate 50% solution | 30,00 | 40,00 | 35,00 | 40,00 |
| Dicaprylyl Ether | 10,00 | 10,00 | 8,00 | 9,00 |
| Glycerin 86% | 5,00 | 3,00 | 5,00 | 3,00 |
| Beheneth-10 | 3,30 | 4,00 | 3,50 | 4,00 |
| Cetearyl Isononanoate | - | - | 4,00 | 5,00 |
| Hexyldecanol / Hexyldecyl Laurate | 3,00 | 5,00 | - | - |
| Parfum | 1,00 | 0,80 | 1,20 | 1,00 |
| Plant Extract | 0,20 | - | 0,50 | - |
| Polysorbate 20 / Linoleic Acid | 0,20 | 0,20 | 0,50 | - |
| Allantoin | 0,10 | - | - | 0,20 |
| Protectate MOD 2 | 0,30 | 0,50 | - | - |
| Protectate HR | - | - | 0,20 | - |
| Aqua | ad 100 | ad 100 | ad 100 | ad 100 |

### 13. Klare Antitranspirant-Gele

| | **1** | **2** | **3** | **4** |
|---|---|---|---|---|
| Propylene Glycol 1,2 | 18,00 | 23,00 | 18,00 | 20,00 |
| Aluminum Chlorohydrate 50% solution | 40,00 | 40,00 | 40,00 | 40,00 |
| Cyclopentasiloxane | 14,20 | 14,20 | 14,20 | 14,20 |
| Alcohol Denat. | 5,00 | 10,00 | 8,00 | 10,00 |
| BIS-PEG/PPG-14/14 Dimethicone | 3,50 | 2,50 | 3,20 | 3,00 |
| Parfum | 0,60 | 0,60 | 1,00 | 1,30 |
| Plant Extract | 0,50 | - | - | - |
| Allantoin | - | 0,10 | - | - |
| 2-Ethylhexylglycerin | 0,30 | - | - | - |
| Protectate MOD 2 | 0,30 | 0,50 | - | - |
| Protectate HR | - | - | 0,20 | - |
| Aqua | ad 100 | ad 100 | ad 100 | ad 100 |

Um klare Gele zu erzielen, ist der Brechungsindex der Wasserphase dem Brechungsindex der Ölphase anzupassen. Wasser bzw. Propylenglycol dienen als Variable.

### 14. Klare Deodorant-Gele

| | **1** | **2** | **3** | **4** |
|---|---|---|---|---|
| Alcohol Denat. | 30,00 | 40,00 | 50,00 | 60,00 |
| Ceteareth-12 | 1,50 | - | 2,00 | - |
| Ceteareth-30 | 2,50 | - | 2,00 | - |
| PEG-40 Hydrogenated Castor Oil | - | 3,00 | - | 2,00 |
| Carbomer | 0,30 | 0,50 | 0,80 | 1,00 |
| Neutralisation Agent | q.s.* | q.s.* | q.s.* | q.s.* |
| Parfum | 0,60 | 0,60 | 1,00 | 1,30 |
| Plant Extract | 0,50 | - | 0,20 | - |
| Ethylhexylglycerin | - | 0,30 | - | - |
| Protectate MOD 2 | 0,30 | 0,50 | - | - |
| Protectate HR | - | - | 0,40 | - |
| Phenoxyethanol | - | 1,00 | - | - |
| Aqua | ad 100 | ad 100 | ad 100 | ad 100 |

Das Verdickungsmittel (Carbomer) ist mit einem geeigneten Neutralisierungsmittel (Triethanolamin, 2-Amino-2-methylpropanol-1 (AMP), Natriumhydroxid, Lithiumhydroxid) auf den gewünschten pH-Wert einzustellen.

### 15. Antitranspirant-Creme

| | **1** | **2** | **3** | **4** |
|---|---|---|---|---|
| Aluminum Chlorohydrate 50% solution | 40,00 | 40,00 | 35,00 | 45,00 |
| Glyceryl Stearate | 5,00 | 4,50 | 5,50 | 6,00 |
| Cetyl Alcohol | 2,00 | - | 3,00 | 1,50 |
| Behenyl Alcohol | 1,50 | 4,00 | 3,50 | 5,00 |
| Dimethicone | 2,00 | 1,50 | 2,50 | 3,00 |
| Ceteareth-12 | 1,50 | 2,00 | 2,50 | 1,30 |
| Ceteareth-20 | 1,50 | 2,00 | 2,50 | 1,30 |
| Hexyldecanol / Hexyldecyl Laurate | 3,00 | 4,00 | 2,50 | 2,40 |
| Cyclopentasiloxane | 1,50 | 3,00 | 2,00 | 1,00 |
| Plant Extract | 0,20 | 0,50 | - | - |
| Tocopheryl Acetate | 0,05 | 0,25 | - | - |
| Parfum | 0,80 | 1,00 | 1,50 | 2,00 |
| Allantoin | 0,10 | 0,10 | - | - |
| Preservative System | 0,05 | 0,50 | 0,50 | 0,50 |
| 2-Ethylhexylglycerin | 0,30 | - | - | - |
| Protectate MOD 2 | 0,30 | 0,50 | - | - |
| Protectate HR | - | - | 0,20 | - |
| Aqua | ad 100 | ad 100 | ad 100 | ad 100 |

### 16. Tränklösungen für Deodorant-Tücher

| | **1** | **2** | **3** | **4** |
|---|---|---|---|---|
| Alcohol Denat. | 50,00 | 55,00 | 60,00 | 40,00 |
| Triethylcitrate | 2,50 | 3,50 | 4,00 | 3,00 |
| PEG-40 Hydrogenated Castor Oil | 1,00 | 0,50 | 0,50 | 2,00 |
| 2-Ethylhexylglycerin | 0,10 | 0,30 | - | - |
| Tocopheryl Acetate | 0,05 | 0,20 | 0,10 | - |
| Benzophenone-2 | 0,01 | 0,01 | 0,01 | 0,05 |
| Colours approved for Cosmetics | 0,0001 | 0,0005 | 0,0010 | - |
| Parfum | 0,80 | 1,00 | 2,00 | 1,50 |
| Protectate MOD 2 | 0,30 | 0,50 | - | - |
| Protectate HR | - | - | 0,20 | 0,60 |
| Phenoxyethanol | - | 1,00 | - | - |
| Aqua | ad 100 | ad 100 | ad 100 | ad 100 |

### 17. Tränklösungen (PIT-Emulsionen) für Antitranspirant-Tücher

| | **1** | **2** | **3** | **4** |
|---|---|---|---|---|
| Aluminum chlorohydrate 50% solution | 30,00 | 40,00 | 35,00 | 40,00 |
| Dicaprylyl Ether | 10,00 | 10,00 | 8,00 | 9,00 |
| Glycerin 86% | 5,00 | 3,00 | 5,00 | 3,00 |
| Beheneth-10 | 3,30 | 4,00 | 3,50 | 4,00 |
| Cetearyl Isononanoate | - | - | 4,00 | 5,00 |
| Hexyldecanol / Hexyldecyl Laurate | 3,00 | 5,00 | - | - |
| Parfum | 1,00 | 0,80 | 1,20 | 1,00 |
| Plant Extract | 0,20 | - | 0,50 | - |
| Polysorbate 20 / Linoleic Acid | 0,20 | 0,20 | 0,50 | - |
| Allantoin | 0,10 | - | - | 0,20 |
| 2-Ethylhexylglycerin | 0,30 | - | - | - |
| Protectate MOD 2 | 0,30 | 0,50 | - | - |
| Protectate HR | - | - | 0,20 | - |
| Preservative System | 0,50 | 0,20 | 1,00 | 0,50 |
| Aqua | ad 100 | ad 100 | ad 100 | ad 100 |

## Patentansprüche

1. Verwendung von mindestens einer Substanz, die an der Körpergeruchsbildung beteiligte Gram-positive anaerobe Kokken inhibiert, in einer kosmetischen Deodorant- oder Antitranspirant-Zusammensetzung zur Verringerung des Körpergeruchs.

2. Verwendung gemäß Anspruch 1, **dadurch gekennzeichnet, dass** die an der Körpergeruchsbildung beteiligten Gram-positiven anaeroben Kokken ausgewählt sind aus Bakterien der Gattung *Peptostreptococcus.*

3. Verwendung gemäß Anspruch 1 oder 2, **dadurch gekennzeichnet, dass** die an der Körpergeruchsbildung beteiligte Gram-positive anaerobe Kokken und insbesondere Bakterien der Gattung *Peptostreptococcus* inhibierende(n) Substanz(en) ausgewählt ist/sind aus funktionellen C₂-C₁₂-Carbonsäuren und deren physiologisch verträglichen Metallsalzen, einfach ungesättigten cis-C₄-C₃₀-Dicarbonsäuren, Derivaten von C₃-C₉-Polyolen, Phenolderivaten, primären Alkanolen mit einer Alkylkettenlänge von C₃ - C₈ und einem Benzyl- oder Phenylsubstituenten, Aluminiumchlorohydrat, Aluminiumzirkoniumchlorohydrat und den Riechstoffgemischen Protectate HR und Protectate MOD 2 der Firma Symrise sowie Mischungen dieser Substanzen.

4. Verwendung gemäß Anspruch 3, **dadurch gekennzeichnet, dass** die funktionellen C₂-C₁₂-Carbonsäuren ausgewählt sind aus C₂-C₁₂-Hydroxycarbonsäuren sowie den physiologisch verträglichen Metallsalzen dieser Säuren.

5. Verwendung gemäß einem der Ansprüche 3 oder 4, **dadurch gekennzeichnet, dass** die physiologisch verträglichen Metallsalze ausgewählt sind aus den Alkalimetall-, Magnesium-, Calcium-, Aluminium-, Zink- und Mangan-Salzen.

6. Verwendung gemäß Anspruch 3, **dadurch gekennzeichnet, dass** die einfach ungesättigten cis-C₄-C₃₀-Dicarbonsäuren ausgewählt sind aus 5(Z)-Decendicarbonsäure, 6(Z)-Dodecendicarbonsäure, 7(Z)-Tetradecendicarbonsäure, 8(Z)-Hexadecendicarbonsäure, 9(Z)-Octadecendicarbonsäure und 10(Z)-Eicosendicarbonsäure.

7. Verwendung gemäß Anspruch 3, **dadurch gekennzeichnet, dass** die Derivate von C₃-C₉-Polyolen ausgewählt sind aus Monoalkyl-C₃-C₉-Polyolethern und C₈₋₂₂-Alkylmono- und -oligoglucosiden der allgemeinen Formel RO-(G)ₓ, wobei R für eine C₈-C₂₂-Alkylgruppe, G für Glucose sowie x für eine rationale Zahl von 1 - 8 steht.

8. Verwendung gemäß Anspruch 7, **dadurch gekennzeichnet, dass** die Monoalkyl-C₃-C₉-Polyolether ausgewählt sind aus α-Monohexylglycerinether, α-Mono-(2-ethylhexyl)glycerinether, α-Mono-(2-ethyloctyl)glycerinether und α-Mono-(2-ethyldecyl)glycerinether.

9. Verwendung gemäß Anspruch 3, **dadurch gekennzeichnet, dass** die Phenolderivate ausgewählt sind aus Phenoxyethanol, 4-Methoxybenzylalkohol (para-Anisalkohol) und 5-Methyl-2-isopropylphenol (Thymol) sowie Mischungen dieser Substanzen.

10. Verwendung gemäß Anspruch 3, **dadurch gekennzeichnet, dass** die primären Alkanole mit einer Alkylkettenlänge von C₃ - C₈ und einem Benzyl- oder Phenylsubstituenten ausgewählt sind aus 2-Methyl-5-phenylpentan-1-ol und 2-Benzylheptan-1-ol.

11. Verwendung gemäß einem der Patentansprüche 1 - 3, **dadurch gekennzeichnet, dass** die an der Körpergeruchsbildung beteiligte Gram-positive anaerobe Kokken und insbesondere Bakterien der Gattung *Peptostreptococcus* inhibierenden Substanzen ausgewählt sind aus Mischungen aus 0,1 - 2,0 Gew.-% α-Mono-(2-ethylhexyl)glycerinether, 0,1 - 2,5 Gew.-% Phenoxyethanol und 0,05 - 1,0 Gew.-% Protectate MOD 2 der Firma Symrise, wobei alle Mengenangaben auf das Gewicht der gesamten kosmetischen Zusammensetzung bezogen sind.

12. Verwendung gemäß Anspruch 11, **dadurch gekennzeichnet, dass** die Mischungen zusätzlich 0,1 - 6 Gew.-% Triethylcitrat, bezogen auf das Gewicht der gesamten kosmetischen Zusammensetzung enthalten.

13. Verwendung gemäß einem der vorhergehenden Ansprüche, **dadurch gekennzeichnet, dass** mindestens eine Bakterien der Gattung *Peptostreptococcus* inhibierende Substanz *Anaerococcus octavius* (auch als *Peptostreptococcus octavius* bezeichnet) inhibiert.

14. Verfahren zur Verringerung von Körpergeruch mittels Inhibierung von an der Körpergeruchsbildung beteiligten Gram-positiven anaeroben Kokken, insbesondere Bakterien der Gattung *Peptostreptococcus,* auf der Haut, **dadurch gekennzeichnet, dass** eine kosmetische Deodorant- oder Antitranspirant-Zusammensetzung, enthaltend mindestens eine an der Körpergeruchsbildung beteiligte Gram-positive anaerobe Kokken, insbesondere Bakterien der Gattung *Peptostreptococcus* inhibierende Substanz auf die Haut, insbesondere auf die Haut der Achselhöhlen, aufgetragen wird.

15. Verfahren zur Verringerung von Körpergeruch nach Anspruch 14, **dadurch gekennzeichnet, dass** die an der Körpergeruchsbildung beteiligte Gram-positive anaerobe Kokken, insbesondere Bakterien der Gattung *Peptostreptococcus* inhibierende(n) Substanz(en) ausgewählt ist/sind aus funktionellen C₂-C₁₂-Carbonsäuren und deren physiologisch verträglichen Metallsalzen, einfach ungesättigten cis-C₄-C₃₀-Dicarbonsäuren, Derivaten von C₃-C₉-Polyolen, Phenolderivaten, primären Alkanolen mit einer Alkylkettenlänge von C₃ - C₈ und einem Benzyl- oder Phenylsubstituenten, Aluminiumchlorohydrat, Aluminiumzirkoniumchlorohydrat und den Riechstoffgemischen Protectate HR und Protectate MOD 2 der Firma Symrise sowie Mischungen dieser Substanzen.

16. Verfahren zur Verringerung von Körpergeruch gemäß Anspruch 14 oder 15, **dadurch gekennzeichnet, dass** die an der Körpergeruchsbildung beteiligte Gram-positive anaerobe Kokken, insbesondere Bakterien der Gattung *Peptostreptococcus* inhibierende(n) Substanz(en) hinsichtlich ihrer Menge und/oder ihrer Art geschlechtsspezifisch eingesetzt werden.

17. Verfahren zur Verringerung von Körpergeruch gemäß Anspruch 16, **dadurch gekennzeichnet, dass** die an der Körpergeruchsbildung beteiligte Gram-positive anaerobe Kokken, insbesondere Bakterien der Gattung *Peptostreptococcus* inhibierende(n) Substanz(en) zur Verringerung von Körpergeruch beim Mann eingesetzt wird/werden.
